(19) Europäisches Patentamt
European Patent Office
Office européen des brevets

(11) Publication number: 0 182 546
A2

(12) # EUROPEAN PATENT APPLICATION

(21) Application number: 85308070.3

(22) Date of filing: 06.11.85

(51) Int. Cl.⁴: G 01 N 33/541

(30) Priority: 12.11.84 GB 8428487

(43) Date of publication of application: 28.05.86
Bulletin 86/22

(84) Designated Contracting States: BE CH DE FR GB IT LI NL SE

(71) Applicant: POLYCLONAL ANTIBODIES LIMITED,
14/15 Newbury Street, London EC1A 7HU (GB)

(72) Inventor: Landon, John, 85 West Street,
Harrow-on-the-Hill Middlesex (GB)

(74) Representative: Wain, Christopher Paul et al, A.A. THORNTON & CO. Northumberland House 303-306 High Holborn, London WC1V 7LE (GB)

(54) **Immunoassays.**

⑤ In the immunoassay of an antigen using the second antibody technique, the second antibody is raised against the Fc component only of the first antibody. This reduces, or reduces the risk of, interference in the primary reaction by the second antibody. In a modified «two-site» immunoassay, a second antibody is used, directed against the Fc component of the unlabelled first antibody, to insolubilise the first antibody. Single reagent immunoassays may be effected using the modified second antibody technique of the invention.

EP 0 182 546 A2

# IMMUNOASSAYS

This invention relates to immunoassays.

Immunoassays depend upon the reversible and non-covalent binding of an antigen (Ag), which is the analyte to be measured by its specific antibodies (Ab), to form a complex (Ag:Ab), in accordance with the reaction:

$$\underset{\text{(free fraction)}}{Ag + Ab} \rightleftharpoons \underset{\text{(bound fraction)}}{Ag:Ab}$$

At equilibrium, some of the free reactants will be combining to form more complex, and some of the complex will be dissociating to give the free reactants.

In order to enable, or improve the accuracy of, quantitation in immunoassays, it is known to use a labelled reagent. Among the labels commonly used are radioisotopes, enzymes and fluorophors.

In many immunoassays, it is necessary to separate the bound fraction from the free fraction once equilibrium has been established. A large number of physicochemical, immunological and other means of separating the antibody-bound and free fractions have been developed. Among these is the so-called second antibody approach. In accordance with this technique, there is included in the reaction mixture, in addition to the antibody (first antibody) directed against the antigen, another antibody (second

antibody) which is directed against the first antibody. The conditions are such that precipitation of the antigen:first antibody:second antibody complexes occurs, and these can then be separated from the remainder of the reaction mixture in which the free fraction remains.

Whilst this second antibody technique is quite widely and successfully used, it has certain drawbacks. In particular, the second antibody can interfere in the primary immunological reaction between the antigen and the first antibody. An antibody IgG molecule comprises two antigen binding sites (in the Fab components) and another component (Fc) not directly involved in antigen-antibody binding. When an animal is immunised with IgG (i.e. when it is immunised with first antibody IgG to raise the second antibody there-against), populations of antibodies are formed some of which bind to the Fab portion and can thus subsequently interfere in the antigen:first antibody reaction because they are in the vicinity of the hypervariable binding site. Thus, we have found that, in the labelled assay of $T_4$ (thyroxine) using the second antibody technique, there can be impairment of the $^{125}I-T_4$ binding because of interference by the second antibody.

We have now found a way in which this problem can be reduced or overcome. In particular, we have found that second antibody interference can be markedly reduced or even eliminated by using, as the second antibody, an antibody raised not against the whole first antibody IgG but only against the Fc fragment of the first antibody. In this way, the second antibodies so made will bind only to antigenic determinants on the Fc component of the first antibody and will not interfere with the primary antigen: first antibody reaction. Thus, the cause or main cause of interference is removed without loss of the essential function of the second antibody which is to bind with and insolubilise the first antibody.

0182546



In one aspect, the present invention thus provides a method of immunoassay of an antigen wherein the antigen is reacted with a first antibody to form an antigen:antibody complex, and wherein a second antibody is used to bind to the first antibody to form a precipitate therewith, the precipitate being separated out, characterised in that the second antibody is directed only against the Fc component of the first antibody.

The method of the invention is applicable to all second antibody assays, including those using a labelled reagent, in which there is a separation step.

One particular form of immunoassay with which the present invention may be utilised is that known as a "two-site" technique for the assay of large molecules with more than one antigenic determinant. In a conventional "two-site" assay, the sample under assay is first incubated with an excess of an antibody covalently linked to an insoluble support and directed against one antigenic determinant ($SP\text{-}Ab_1$)

$$Ag + \text{excess } SP\text{-}Ab_1 \longrightarrow SP\text{-}Ab_1{:}Ag + Sp\text{-}Ab_1 \quad \text{-----------(i)}$$

After careful washing, excess of a second, isotopically labelled antibody ($Ab_2^*$) directed against another part of the antigen molecule is added

$$SP\text{-}Ab_1 + SP\text{-}Ab_1{:}Ag + \text{excess } Ab_2^* \longrightarrow$$
$$SP\text{-}Ab_1 + SP\text{-}Ab_1{:}Ag{:}Ab_2^* + Ab_2^* \quad \text{------------- (ii)}$$

Following further washes, the counts (in the case of a radiolabel) present in the solid phase fraction are determined and are related directly to the concentration of antigen present in the samples and standards.

In accordance with a preferred aspect of the present invention, insolubilising second antibodies are used instead of the solid phase. Thus, the antibodies $Ab_1$ and $Ab_2^*$ are preferably raised in different species, e.g. rabbit and sheep respectively, and the insolubilising second antibody is raised in sheep and is directed against the Fc fragment of rabbit IgG. It will therefore combine with $Ab_1$, but not with $Ab_2$, and hence will insolubilise the bound $Ab_2^*$ but not the free $Ab_2^*$. Thus, a separation can be effected.

The use, in accordance with the invention, of certain insolubilising second antibodies instead of antibodies covalently linked to a solid phase avoids various problems associated with the use of such a solid phase, including the necessity to prepare the solid phase reagent (which can be difficult and expensive) and the problems of accurately measuring out antibodies attached to a solid phase, for example.

Thus, in a further aspect, the present invention provides a two-site immunoassay of an antigen having more than one antigenic determinant, wherein the antigen is mixed with a first antibody against one of its determinants to form a first complex, the said complex is mixed with excess of a labelled antibody against a second determinant on the antigen to form a second complex, the second complex being insoluble and thus separable from labelled antibody remaining in solution, characterised in that there is also included in the solution an antibody directed against the Fc component only of said first antibody and which insolubilises said first antibody upon binding thereto.

There have been attempts in the past to simplify immunoassays, especially label assays, in particular to make them into "single reagent" assays, that is assays in which the sample under test is mixed with a "single reagent", and the mixture simply treated to reveal the

the assay result. The so-called "single reagent" is, of course, a mixture of all the various reaction components required for the assay (excepting the sample or standard). In theory, single reagent label immunoassays should significantly reduce the complexity of label assays, and increase the speed and precision thereof. Also, they should reduce reagent manufacturing costs and also improve the stability of some isotopically labelled antigens.

However, despite these potential advantages, there have been only a very few proposals hitherto for single reagent label immunoassays and, so far as we are aware, none of these has been very satisfactory. For example, one commercially available kit for the radioimmunoassay of thyroxine ($T_4$) used a single reagent comprising $^{125}I-T_4$, mixed with anti-$T_4$ antiserum and second antibody raised against the entire immunoglobulin G (IgG) of the species used to make the antiserum. However, in conducting the assay, the mixture of sample (or standard) and single reagent had to be incubated for 30 minutes at 56°C, which was not very convenient. Furthermore, the assay gave relatively poor precision (up to 10% coefficients of variation) and high recovery (up to 125%) as compared with a conventional double antibody assay for $T_4$. In another proposed radioimmunoassay (RIA) for $T_4$, it was found that the assay was accurate only if the "single reagent" was made up immediately before use. Again, this is not very convenient and somewhat negates the advantages theoretically achievable by "single reagent" procedures.

We have now found, in accordance with a further aspect of the present invention, by using a second antibody directed against the Fc fragment only of the first antibody, "single reagents" can be made up which are stable and reliable in subsequent use in an assay. Thus, the invention includes a single reagent label immunoassay

for an antigen in which the antigen is mixed with a single reagent mixture and, optionally after the separation step, the label is assayed, characterised in that the single reagent mixture comprises a first antibody against the antigen and a second antibody against the Fc component of the first antibody.

Further, the two-site assay of the invention can also be conducted, in accordance with a further aspect of the invention, as a "single reagent" assay. The "single reagent" mixture for use in such an assay preferably comprises antibody Ab, raised in a first species against a determinant of the Ag under assay; labelled antibody $Ab_2$ raised in a second species against another determinant of the Ag; and antibody $Ag_3$ raised in said second species against the Fc fragment of the IgG of the first species.

There are a number of advantages of being able to conduct the two-site assay as a single reagent assay. There is, firstly, the necessity for only one single reagent addition, and at most one lot of washing prior to isotope counting. Further, by using the insolubilising antibody technique of the second aspect of the invention, non-specific binding is reduced or avoided. This is important because non-specific binding is of particular relevance in such assays since their sensitivity is related directly to the signal to "noise" ratio.

In order that the invention may be more fully understood, the following Examples are given by way of illustration only.

1. Example of a separation immunoassay employing anti-Fc second antibody:fluoroimmunoassay of human serum albumin

Human serum albumin (HSA) was labelled with fluorescein (molar ratio fluorescein:HSA of 2:1) by reaction

with fluorescein isothiocynate according to standard methods. Antiserum to HSA was raised in a sheep by standard procedures. Antisera to sheep IgG and to sheep Fc fragment were raised in rabbits by standard procedures. Assay diluent buffer was sodium phosphate (50 mmol/l pH 7.4) containing bovine serum albumin (5 g/l) and sodium azide (1 g/l). Standard solutions of HSA were prepared in diluent buffer.

Experiments were performed using polystyrene test tubes which also served as cuvettes for measurement of fluorescence in a Perkin-Elmer Model 1000M filter fluorimeter (Kamel et al. (1980) Clin. Chem. 26, 1281-1284).

For the fluoroimmunoassays with conventional reagent addition sequence, working tracer reagent was prepared by diluting the fluorescein-labelled HSA to 2.6 mg/l (based on its protein content) in diluent buffer and working antiserum reagent by diluting the anti-HSA serum by 1,430-fold in diluent buffer.

The assay procedure with conventional second-antibody separation was as follows. To 50 µl of standard was added 200 µl of working tracer reagent followed by 100 µl of working antiserum reagent. After incubation for 1 h at room temperature, 50 µl of normal sheep serum (diluted 1,000-fold in diluent buffer) and 50 µl of second antiserum (either anti-IgG or anti-Fc, each diluted 8-fold in diluent buffer) was added. After further incubation for 2 h at room temperature, the assay mixtures were centrifuged, the supernatants aspirated to waste, and the precipitates re-dissolved by the addition of 1.5 ml of a mixture of methanol, water and 2 mol/l sodium hydroxide (50:50:1 parts by volume). The fluorescence of the re-dissolved precipitates (antibody-bound fraction of the labelled HSA) was measured by placing each test tube into the fluorimeter.

The fluoroimmunoassay standard curves obtained

with the conventional assay procedure and with anti-IgG or anti-Fc second antiserum are shown in Figures 1 and 2, respectively (triangles).

For fluoroimmunoassays employing pre-combined first and second antibodies, mixtures were prepared containing sheep anti-HSA serum (dilution 1,430-fold), normal sheep serum (dilution 2,000-fold) and either rabbit anti-(sheep IgG) serum or rabbit anti-(sheep Fc) serum (each diluted 16-fold). The antiserum mixtures were allowed to equilibrate for 1 h at room temperature before being used in a fluoroimmunoassay according to the following procedure.

To 50 µl of standard was added 200 µl of working tracer reagent followed by 100 µl of the pre-combined antisera mixture, then 100 µl of diluent buffer. After incubation for 3 h at room temperature the immunoassay mixtures were centrifuged, the supernatants aspirated, the precipitates re-dissolved, and fluorescence measured, as before.

Figures 1 and 2 show the standard curves obtained using pre-combined antisera reagents containing anti-IgG or anti-Fc second antiserum, respectively (circles). In the system with anti-IgG second antiserum, pre-combination with first antiserum evidently leads to hindrance of binding of the labelled HSA (Figure 1). When anti-Fc second antiserum is employed (Figure 2) there is no such degradation of assay performance (the slight increase in labelled HSA binding noted in the pre-combined assay system may be attributable to the longer overall time of incubation - 4 h as compared with 2 h in the conventional procedure - of the first and second antisera, leading to more efficient precipitation of the bound fraction).

2. <u>Example of a single reagent immunoradiometric assay employing anti-Fc second antibody: liquid-phase two-site assay of human prolactin</u>

Antisera to human prolactin were raised in sheep and rabbits by standard procedures. Specific anti-prolactin immunoglobulins were isolated from the sheep antiserum by immunoaffinity purification and were radioiodinated with $^{125}I$ by conventional procedures. Antisera to rabbit IgG and to rabbit Fc fragment were raised in sheep by standard procedures. Assay diluent buffer was sodium phosphate (64 mmol/l, pH 7.4) containing polyethylene glycol 6000 (30 g/l), bovine serum albumin (5 g/l), normal rabbit serum (10 ml/l), normal sheep serum (20 ml/l), and thiomersal (100 mg/l). Standard solutions of human prolactin were prepared in horse serum.

The procedure for conventional liquid-phase two-site assay was as follows. To 100 µl of standard was added 100 µl of rabbit anti-prolactin serum (diluted 400-fold in diluent buffer) followed by 100 µl (approx. 100,000 counts/min) of $^{125}I$-labelled sheep anti-prolactin immunoglobulins. After incubation for 3 h at room temperature, 100 µl of sheep anti-(rabbit (IgG) serum (appropriately) diluted in 64 mmol/l sodium phosphate buffer, pH 7.4) was added and after incubation for a further 1 h at room temperature the assay mixtures were centrifuged, the supernatants aspirated to waste and the radioactivity in the precipitates counted. The standard curve obtained is shown in Figure 3.

When the above experiment was repeated employing an appropriate dilution of sheep anti-(rabbit Fc) second antiserum in place of the anti-(rabbit IgG) serum, an identical standard curve was obtained.

The experiment of Figure 3 was repeated employing a mixture of the two specific anti-prolactin reagents that had been prepared several days previously. To 100 µl of standard was added 200 µl ______________________

a mixture of appropriate proportions of rabbit anti-prolactin serum and $^{125}$I-labelled sheep anti-prolactin immunoglobulins. After incubation for 3 h at room temperature, 100 µl of sheep anti-(rabbit IgG) serum was added and after incubation for a further 1 h at room temperature the assay mixtures were centrifuged, supernatants aspirated, and precipitates counted, as before. The standard curve obtained was identical with that of Figure 3. Thus it is possible to combine the two first-antibody reagents, thereby reducing the number of pipetting steps in the assay by one.

An experiment was performed in which the two specific anti-prolactin reagents and sheep anti-(rabbit IgG) second antiserum were mixed in appropriate proportions and stored for several days before use. To 100 µl of standard was added 300 µl of the single reagent mixture of rabbit anti-prolactin serum, $^{125}$I-labelled sheep anti-prolactin immunoglobulins and sheep anti-(rabbit IgG) serum. After incubation for 3 h at room temperature, the mixtures were centrifuged, supernatants aspirated, and precipitates counted, as before. Although two pipetting steps and considerable incubation time were saved, the resulting standard curve (Figure 4; triangles) was poor. It was concluded that the sheep anti-(rabbit IgG) antibodies were interfering with the primary antigen:antibody reaction by binding to the Fab part of the rabbit first antibody and thereby hindering the binding of prolactin.

The above experiment was repeated except that sheep anti-(rabbit Fc) serum was used in place of the anti-(rabbit IgG) serum in the single reagent mixture. The standard curve obtained (Figure 4; circles) was similar to that for the conventional assay procedure (Figure 3), indicating that the anti-Fc second antibodies did not interfere with the primary antigen:antibody reaction. This assay proved simpler and quicker to perform than the

conventional procedure - avoiding two pipetting steps and the need for a second incubation, and with all reagent additions completed at one time.

CLAIMS:

1. A method of immunoassay of an antigen wherein the antigen is reacted with a first antibody to form an antigen:antibody complex, and wherein a second antibody is used to bind to the first antibody to form a precipitate therewith, the precipitate being separated out, characterised in that the second antibody is directed only against the Fc component of the first antibody.

2. A method according to claim 1, wherein the immunoassay is a label assay.

3. A two-site immunoassay of an antigen having more than one antigenic determinant, wherein the antigen is mixed with a first antibody against one of its determinants to form a first complex, the said complex is mixed with excess of a labelled antibody against a second determinant on the antigen to form a second complex, the second complex being insoluble and thus separable from labelled antibody remaining in solution, characterised in that there is also included in the solution a second antibody directed against the Fc component only of said first antibody and which insolubilises said first antibody upon binding thereto.

4. An immunoassay according to claim 3, wherein the first antibody and second antibody are of one animal species and the labelled antibody is of another animal species.

5. An assay according to claim 2,3 or 4, wherein the label is a radioisotope, enzyme or fluorophore.

6. A single reagent label immunoassay for an antigen, in which the antigen is mixed with a single reagent mixture and, optionally after a separation step, the label is assayed, characterised in that the single reagent mixture comprises a first antibody against the antigen and a second antibody against the Fc component of the first antibody.

7. An assay according to claim 6, wherein said antigen has at least two antigenic determinants, and wherein said "single reagent" mixture comprises antibody raised in a first species against a determinant of the antigen under assay; labelled antibody raised in a second species against another determinant of the antigen; and antibody raised in said second species against the Fc fragment of the IgG of the first species.

8. An immunoassay according to claim 6 or 7, wherein the label is a radioisotope, enzyme or fluorophore.

9. A "single reagent" mixture for use in the assay of claim 6, which comprises a first antibody against an antigen, and a second antibody against the Fc component of the first antibody.

10. A "single reagent" mixture for use in the assay of claim 7, which comprises antibody of a first animal species against an antigen, labelled antibody of a second animal species against the antigen, and antibody raised in said first species against the Fc component of the IgG of the first species.

0182546

1/4

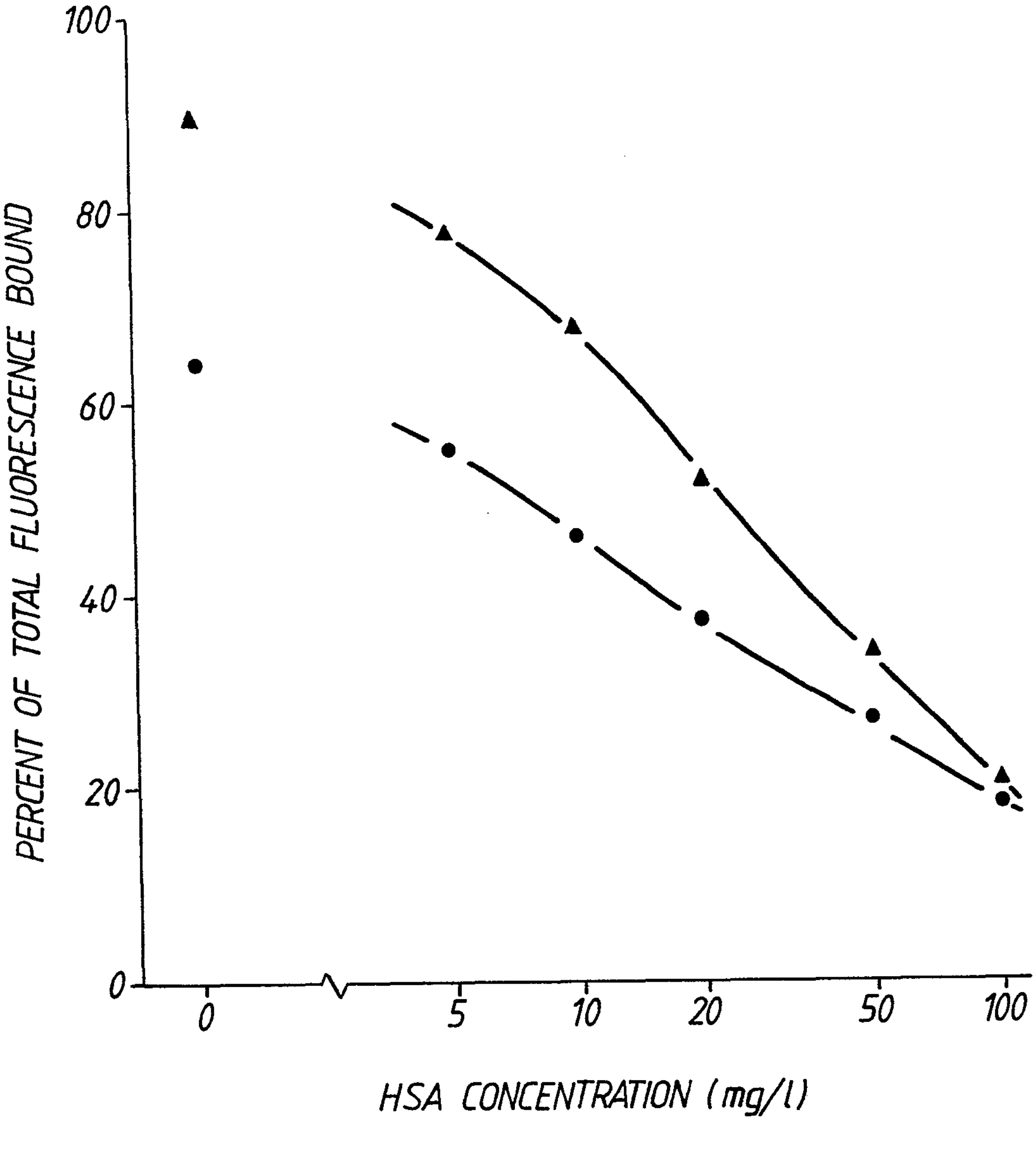


FIG. 1.

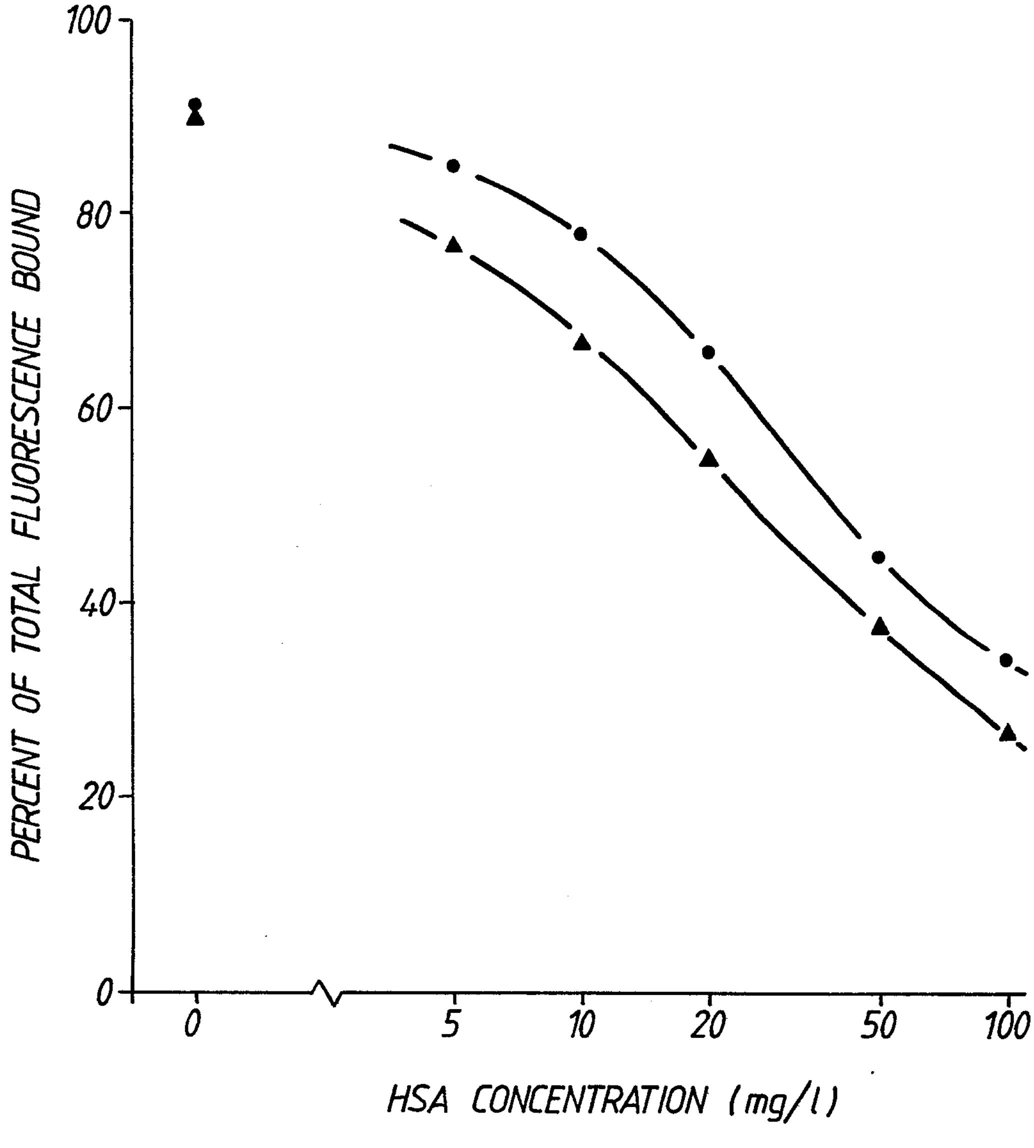

100
80
60
40
20
0
PERCENT OF TOTAL FLUORESCENCE BOUND
0
5
10
20
50
100
HSA CONCENTRATION (mg/l)


FIG. 2.

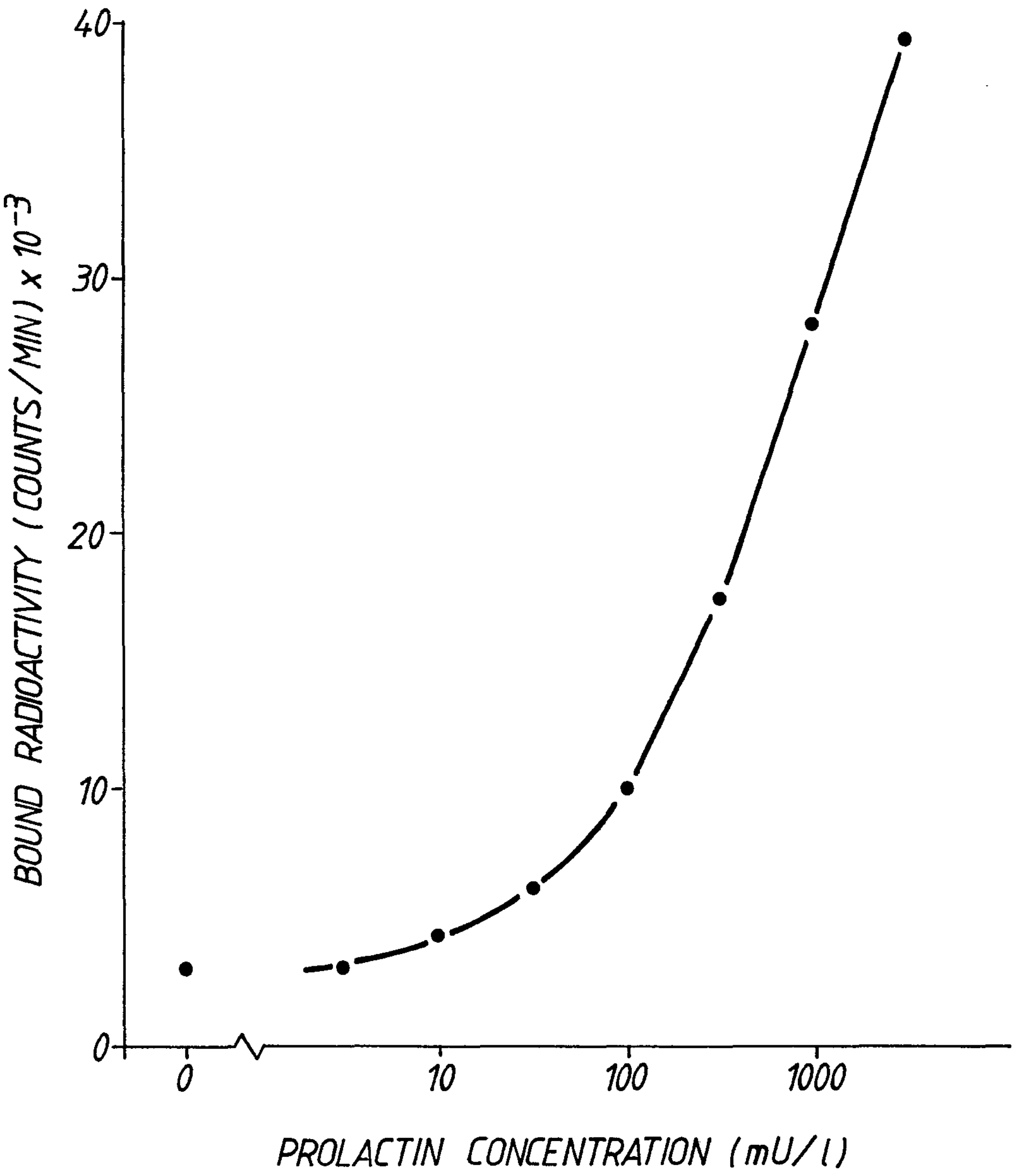
40
30
20
10
0
BOUND RADIOACTIVITY (COUNTS/MIN) x $10^{-3}$
0
10
100
1000
PROLACTIN CONCENTRATION (mU/l)

FIG. 3.

0182546

4/4

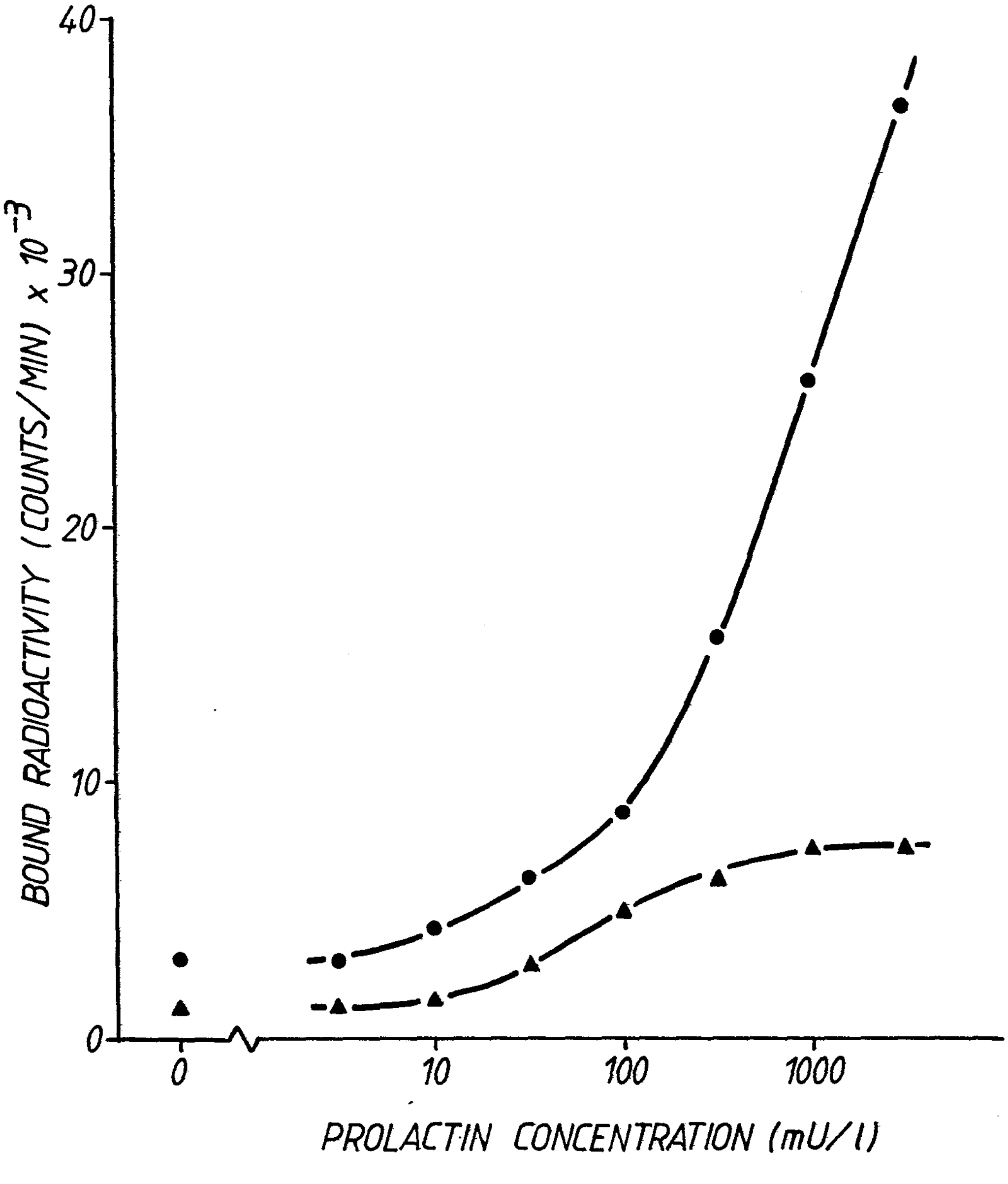


FIG. 4.